# EUROPEAN PATENT SPECIFICATION

(11) **EP 3 281 932 B1**
(45) Date of publication and mention of the grant of the patent: **12.08.2020**
(21) Application number: 16776717.7
(22) Date of filing: 08.04.2016
(51) Int. Cl.: C07C 51/64, C07C 57/76, C07C 67/62, C07C 69/653

(54) **COMPOSITION CONTAINING ACRYLIC ACID DERIVATIVE, AND METHOD FOR STABILIZING ACRYLIC ACID DERIVATIVE**
ZUSAMMENSETZUNG MIT ACRYLSÄUREDERIVAT UND VERFAHREN ZUR STABILISIERUNG VON ACRYLSÄUREDERIVAT
COMPOSITION CONTENANT UN DÉRIVÉ D'ACIDE ACRYLIQUE, ET PROCÉDÉ DE STABILISATION DU DÉRIVÉ D'ACIDE ACRYLIQUE

(30) Priority: 09.04.2015 JP 2015080382; 27.04.2015 JP 2015090688
(43) Date of publication of application: 14.02.2018
(73) Proprietor: Daikin Industries, Ltd., Osaka-shi, Osaka 530-8323 (JP)
(72) Inventor: MATSUURA, Makoto, Osaka-shi, Osaka 530-8323 (JP); YOSHIYAMA, Asako, Osaka-shi, Osaka 530-8323 (JP); KISHIKAWA, Yosuke, Osaka-shi, Osaka 530-8323 (JP)
(74) Representative: Hoffmann Eitle
(86) International application number: PCT/JP2016/061624
(87) International publication number: WO 2016/163551

(56) References cited:
- DE-A1- 2 350 119
- DE-A1- 2 350 119
- GB-A- 1 408 629
- JP-A- S4 993 315
- JP-A- S5 785 337
- JP-A- H02 500 026
- JP-A- H11 255 703
- JP-A- S60 158 136
- JP-A- S62 106 049
- JP-A- 2003 277 319
- US-A- 2 886 494
- US-A- 4 671 857

## Description

### Technical Field

The present invention relates to a composition containing an acrylic acid derivative, and to a method for stabilizing an acrylic acid derivative.

### Background Art

Acrylic acid derivatives are widely used for (1) materials of water absorbing polymers, (2) materials of acrylic resins as a substitute for inorganic glass for use in, for example, window materials for buildings and vehicles, coverings for lighting equipment, lantern signs, road signs, daily necessities, office supplies, crafts and windscreens of watches, and (3) acrylic resin coating materials. Among acrylic acid derivatives, fluorine-containing acrylic acid derivatives are useful as synthetic intermediates of pharmaceuticals (e.g., antibiotics), synthetic intermediates for sheath materials of optical fibers, synthetic intermediates of coating materials, synthetic intermediates of semiconductor resist materials, and monomers of functional polymers.

Examples of known methods for producing an acrylic acid derivative include a method of producing an acrylic acid derivative by oxidizing isobutylene or propylene, and a method of producing an acrylic acid derivative using, for example, ethylene or propyne as a starting material using a transition metal catalyst.

Further, as examples of methods for producing a fluorine-containing acrylic acid derivative, for example, JP-A-2011-001340 discloses a method of reacting a 2-fluoropropionic ester with a nitrogen-bromine-bond-containing brominating agent in the presence of a radical initiator, and JP-A-2012-530756 discloses a process for converting a 3-halo-2-fluoropropionic acid derivative to a substituted 2-fluoroacrylic acid derivative in the presence of at least one kind of base and at least one kind of polymerization inhibitor.

GB-A-1408629 discloses a method for producing α-chloroacrylic chloride by reacting α-chloroacrylic acid with oxalyl chloride, preferably in the presence of a N-substituted carboxylic acid amide as a reaction promoter.

DE-A-23 50 119 describes the manufacture of 2-chloroacylic chloride by reacting 2-chloroacylic acid with oxalyl chloride in the presence of dialkylformamide, oxalic acid and a polymerization inhibitor at a temperature of 10-60°C.

US 4,671,857 relates to a process for separating methacrylic acid from isobutyric acid, comprising distilling a mixture of methacrylic acid, isobutyric acid sand a third component selected from methyl methacrylate, methyl isobutyrate and dimethylformamide in an a mount sufficient to increase the separation efficiency of the distillation process.

Us 2,886,494 defines a composition comprising a liquid monomeric α-haloacrylic ester of an alcohol, such as a lower alkyl ester of α-chloroacrylic acid, and a polymerization inhibiting amount of a 1,4-diamino anthraquinone compound.

### Summary of Invention

### Technical Problem

Since an acrylic acid derivative contains active unsaturated bond due to its structure, it is unstable against external stimuli such as heat, light, and oxygen, and may easily change into an oligomer or a polymer by a polymerization reaction. Therefore, a method for stabilizing an acrylic acid derivative, and a composition containing an acrylic acid derivative in which the acrylic acid derivative is stabilized, have been in demand.

An object of the present invention is to provide a method for stabilizing an acrylic acid derivative, and a composition containing an acrylic acid derivative in which the acrylic acid derivative is stabilized.

### Solution to Problem

The inventors of the present invention conducted extensive research and found that the above problem can be solved by a composition as described herein. Thus, the present invention provides a composition (also referred to as "the present composition" hereinafter) comprising:
(A) an acrylic acid derivative of formula (I): 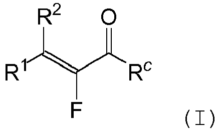 wherein, R¹ and R² each independently are H, halogen, alkyl, fluoroalkyl or optionally substituted aryl; and R*^{c}* is F, Br, I, or -OR³ wherein R³ is H, alkyl, fluoroalkyl or optionally substituted aryl; and
(B) amide,
   wherein the content of the acrylic acid derivative (A) in the composition is ≥ 30% (w/w).

Also, the present invention provides a method (also referred to as "the present method" hereinafter) for stabilizing
(A) an acrylic acid derivative of formula (I): 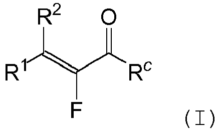 wherein, R¹ and R² each independently are H, halogen, alkyl, fluoroalkyl or optionally substituted aryl; and R*^{c}* is F, Br, I, or -OR³ wherein R³ is H, alkyl, fluoroalkyl or optionally substituted aryl;
   comprising making the acrylic acid derivative of formula (I) coexist with amide.

Preferred embodiments of the invention are as defined in the appended dependent claims and/or in the following detailed description.

### Advantageous Effects of Invention

The present composition contains an acrylic acid derivative; in the composition, the acrylic acid derivative is stabilized. The present method stabilizes an acrylic acid derivative.

### Description of Embodiments

### Terms

The symbols and the abbreviations in this specification are to be interpreted as having the general meanings in the related technical field to which the present invention pertains, according to the context of this specification, unless otherwise specified.

"Room temperature" means a temperature in a range of 10-40°C.

The term "comprise/contain" is intended to mean both "consist essentially of" and "consist of".

"Stabilization" of an acrylic acid derivative refers to preventing an acrylic acid derivative from changing into a different substance, such as a polymer.

"Alkyl" (the term "alkyl" encompasses the "alkyl" moiety in e.g. "fluoroalkyl") may be a cyclic, linear, or branched alkyl, and "alkyl" may be, for example, a C₁₋₂₀, C₁₋₁₂, C₁₋₆, C₁₋₄, or C₁₋₃ alkyl.

Specific examples of "alkyl" include linear or branched alkyl, such as methyl, ethyl, propyl, isopropyl, butyl, isobutyl, sec-butyl, tert-butyl, pentyl, neopentyl and hexyl, and C₃₋₆ cyclic alkyl (cycloalkyl), such as cyclopropyl, cyclobutyl, cyclopentyl and cyclohexyl.

"Fluoroalkyl" refers to an alkyl in which at least one hydrogen is replaced by fluorine. The number of fluorines in the "fluoroalkyl" may be one or more (the maximum replaceable number from 1; e.g., 1-3, 1-6, or 1-12). Tthe "fluoroalkyl" may be a linear or branched fluoroalkyl. Also, the "fluoroalkyl" encompasses perfluoroalkyl, i.e. alkyl in which all of the hydrogens are replaced by fluorines. Examples of "fluoroalkyl" include C₁₋₂₀, C₁₋₁₂, C₁₋₆, C₁₋₄, and C₁₋₃ fluoroalkyls.

Specific examples of "fluoroalkyl" include fluoromethyl, difluoromethyl, trifluoromethyl, 2,2,2-trifluoroethyl, pentafluoroethyl, tetrafluoropropyl (e.g., HCF₂CF₂CH₂-), hexafluoropropyl (e.g., (CF₃)₂CH-), nonafluorobutyl, octafluoropentyl (e.g., HCF₂CF₂CF₂CF₂CH₂-), and tridecafluorohexyl.

Examples of "aryl" include phenyl and naphthyl.

Examples of "halogen" include fluorine, chlorine, bromine, and iodine.

The "alkoxy" is an alkyl-O-group.

Examples of "acyl" include alkanoyl (i.e., alkyl-CO-group).

Examples of "ester" include alkylcarbonyloxy (i.e., alkyl-CO-O-group), and alkoxycarbonyl (i.e., alkyl-O-CO-group).

### Composition

The present composition comprises:
(A) an acrylic acid derivative of formula (I): 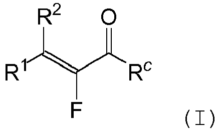 wherein, R¹ and R² each independently are H, halogen, alkyl, fluoroalkyl or optionally substituted aryl; and R*^{c}* is F, Br, I, or -OR³ wherein R³ is H, alkyl, fluoroalkyl or optionally substituted aryl; and
(B) amide,
   wherein the content of the acrylic acid derivative (A) in the composition is ≥ 30% (w/w).

### Acrylic Acid Derivative (A)

Each symbol in Formula (1) representing acrylic acid derivative (A) is explained below.

Preferable examples of the substituents of the "optionally substituted aryl" represented by R¹, R², and R³ include fluorine, alkyl, alkoxy, acyl, ester, cyano, nitro, and fluoroalkyl. More preferable examples include fluorine.

The number of "the substituents" is preferably 0 (i.e., unsubstituted), 1, 2, or 3.

R¹ is preferably H, C₁₋₂₀ (preferably C₁₋₁₂, more preferably C₁₋₆, further preferably C₁₋₄, further more preferably C₁₋₃, particularly preferably C₁ or C₂) alkyl, or C₁₋₂₀ fluoroalkyl, and more preferably H.

R² is preferably H, C₁₋₂₀ (preferably C₁₋₁₂, more preferably C₁₋₆, further preferably C₁₋₄, further more preferably C₁₋₃, particularly preferably C₁ or C₂) alkyl, or C₁₋₂₀ (preferably C₁₋₁₂, more preferably C₁₋₆, further preferably C₁₋₄, further more preferably C₁₋₃, particularly preferably C₁ or C₂) fluoroalkyl, and more preferably H.

The halogen represented by R^{c} is preferably F.

R^{c} is preferably -OR³, and R³ is preferably C₁₋₂₀ (preferably C₁₋₁₂, more preferably C₁₋₆, further preferably C₁₋₄, further more preferably C₁₋₃, particularly preferably C₁ or C₂) linear alkyl.

In Formula (I), preferably,
R¹ is H;
R² is H;
R^{c} is -OR³, and
R³ is methyl or ethyl (more preferably methyl).

The present composition may comprise one or more kinds of acrylic acid derivative (A); however, it preferably comprises only one kind of acrylic acid derivative (A).

Acrylic acid derivative (A) used in the present invention may be produced by a known method or a similar method thereof, or may be obtained from commercial suppliers.

When R^{c} in Formula (I) is -OR³, the acrylic acid derivative (A) may be produced, for example, through the production methods disclosed in International Publication No. WO 2014/034906, JP-A-2014-24755 or US 3,262,968, or similar methods thereof.

When R^{c} in Formula (I) is F, Br or I, the acrylic acid derivative (A) may be produced, for example, through the production methods disclosed in JP-A-1985-078940 or JP-A-1986-085345, or similar methods thereof.

The content of acrylic acid derivative (A) in the present composition is ≥ 30%(w/w). Generally, when the concentration of acrylic acid derivative (A) is high, unintended polymerization reaction more easily occurs. However, in the present composition, even when the content of acrylic acid derivative (A) is high, acrylic acid derivative (A) is stable. Further, the content of acrylic acid derivative (A) in the present composition is preferably ≥ 40%(w/w), ≥ 50%(w/w), ≥ 60%(w/w), ≥ 70%(w/w), ≥ 80% (w/w) or ≥ 90% (w/w) . The upper limit of the content of acrylic acid derivative (A) in the present composition is, for example, but not particularly limited to, 98%(w/w), 95%(w/w), or 90%(w/w). However, as it would be obvious to a person skilled in the art, the upper limit may be limited depending on the amount of amide (B) contained in the present composition.

### Amide (B)

Examples of amide (B) contained in the present composition include N,N-dimethylformamide (DMF), N,N-dimethylacetamide (DMAC), N-methylpyrrolidone, 1,3-dimethyl-2-imidazolidinone, N,N-dimethylacrylamide, N,N-dimethylacetoacetamide, N,N-diethylformamide, and N,N-diethylacetamide.

Amide (B) contained in the present composition is preferably represented by the formula R¹⁰R¹¹-N-CO-R¹² (wherein R¹⁰ and R¹¹ are C₁₋₃ alkyl, and R¹² are H or C₁₋₃ alkyl) . The amide (B) contained in the present composition is preferably C₃₋₈ amide, and particularly preferably N,N-dimethylformamide or N,N-dimethylacetamide. In the present invention, amide (B) may be a single kind of amide (B) or a combination of two or more kinds.

The lower limit of the content of amide (B) in the present composition is preferably 0.01%(w/w), 0.05%(w/w), 0.1%(w/w), 0.5%(w/w), 1.0%(w/w), more preferably 3.0%(w/w), and further preferably 5.0%(w/w).

In accomplishing the stabilization of acrylic acid derivative (A), the upper limit of the content of amide (B) in the present composition is not particularly limited; however, using amide (B) in an amount more than the amount ensuring the desired stabilization of acrylic acid derivative (A) is a disadvantage in terms of cost. Therefore, the upper limit is generally, for example, 50%(w/w), 40%(w/w), 30%(w/w), 20%(w/w), or 10% (w/w) .

The content of amide (B) in the present composition is preferably 0.01-50%(w/w), more preferably 1.0-40%(w/w), further preferably 5.0-30%(w/w), further more preferably 1-3%(w/w).

In the present composition, the lower limit of the ratio of amide (B) to acrylic acid derivative (A) [(B)/(A)] is preferably 0.01%(w/w) or 0.05%(w/w), more preferably 0.1%(w/w), further preferably 0.5%(w/w), further more preferably 1.0%(w/w), particularly preferably 3.0%, more particularly preferably 5.0%(w/w).

In accomplishing the stabilization of acrylic acid derivative (A), the upper limit of the ratio of amide (B) to acrylic acid derivative (A) [(B)/(A)] is not particularly limited; however, using amide (B) in an amount more than the amount ensuring the desired stabilization of acrylic acid derivative (A) is a disadvantage in terms of cost. Therefore, the upper limit of the ratio [(B)/(A)] is generally, for example, 200%(w/w), 190%(w/w), 170%(w/w), 150%(w/w), 100%(w/w), 70%(w/w), 50%(w/w), 40%(w/w), or 30%(w/w).

In the present composition, the ratio [(B)/(A)] is preferably 0.01-200%(w/w), more preferably 0.1-190%(w/w), further preferably 1-170%(w/w), further more preferably 3-50%(w/w), and particularly preferably 5-50%(w/w).

### Optional Components

The present composition may contain optional components in addition to acrylic acid derivative (A) and amide (B). The optional components may be impurities that coexist with acrylic acid derivative (A) or amide (B) prepared for the production of the present composition.

Examples of the optional components include water and organic solvents.

In the present composition, since acrylic acid derivative (A) is stabilized by amide (B), the significance in using a polymerization inhibitor for the purpose of stabilizing acrylic acid derivative (A) is small; however, the present composition may contain a polymerization inhibitor as an optional component.

As a method for preventing unintended polymerization reaction, a method of using a polymerization inhibitor, such as the polymerization inhibitor disclosed in JP-A-2012-530756, has been known. However, acrylic acid derivative (A) may be exposed to various conditions, for example, upon storage or at the time of use. Since the boiling points of versatile polymerization inhibitors greatly differ from that of an acrylic acid derivative, it is often difficult to make them coexist with an acrylic acid derivative. In this case, the polymerization inhibitors cannot fully exhibit the function.

### Stability of the Present Composition

In the present composition, acrylic acid derivative (A) is stabilized. More specifically, acrylic acid derivative (A) contained in the present composition has high stability. For example, acrylic acid derivative (A) is prevented from changing into a polymer, compared with a case in which acrylic acid derivative (A) does not coexist with amide (B).

The change of acrylic acid derivative (A) into a different substance may be analyzed, for example, using NMR analysis. Further, for example, the change of acrylic acid derivative into a polymer may be easily detected by observation of a change of a colorless transparent solution of acrylic acid derivative into a solid.

In the present composition, acrylic acid derivative (A) is stabilized by the coexistence with amide (B).

A method for making the acrylic acid derivative coexist with a polymerization inhibitor has been known as a means for stabilizing an acrylic acid derivative. However, since acrylic acid derivative (A) may be exposed to various conditions, for example, upon storage or at the time of use, the coexistence of a polymerization inhibitor with an acrylic acid derivative may be difficult in some cases. In this case, the polymerization inhibitor cannot fully exhibit its function.

In contrast, since amide (B) may have a boiling point similar to that of acrylic acid derivative (A), it is easy to make amide (B) coexist with acrylic acid derivative (A). Therefore, acrylic acid derivative (A) in the composition of the present invention is stable under various conditions.

### Production Method

The present composition may be produced by mixing acrylic acid derivative (A), amide (B), and optional components using, for example, a usual method such as stirring. Some or all of amide (B) may be contained as an impurity or an additive in acrylic acid derivative (A) prepared for the production of the present composition.

### Method for Stabilizing Acrylic Acid Derivative (A)

The method for stabilizing the present acrylic acid derivative (A) of Formula (I) comprises making acrylic acid derivative (A) coexist with amide (amide (B)). This method is not particularly limited. Examples of the method include:
[1] a method of mixing acrylic acid derivative (A) and amide (B);
[2] a method of producing amide (B) in a system containing acrylic acid derivative (A);
[3] a method of producing acrylic acid derivative (A) in a system containing amide (B); and
[4] a method of individually producing acrylic acid derivative (A) and amide (B) in a single system.

The same explanation of acrylic acid derivative (A) and of amide (B) as that regarding the present composition can be applied to acrylic acid derivative (A) and amide (B) used in the present method for stabilizing acrylic acid derivative (A).

In the present method, preferably, amide (B) is used at a predetermined ratio relative to acrylic acid derivative (A), as described above regarding the present composition.

The details of the present method including the above matters can be understood from the above explanation regarding the present composition.

### Examples

The present invention is described below in more detail with reference to Examples.

### Example 1

2-fluoroacryloyl fluoride purified by distillation was prepared. 0.23 g of N,N-dimethylacetamide and 9.92 g of 2-fluoroacryloyl fluoride were mixed, thereby preparing a sample composition. 9.9 g of 2-fluoroacryloyl fluoride purified by distillation was used as a control. They were both transparent liquids when prepared.

The sample composition, and 2-fluoroacryloyl fluoride as a control were each placed individual sample bottles. Each bottle was closed with a cap and allowed to stand for a day at room temperature. Thereafter, the characteristics of the two samples were observed. The results revealed that a solid was observed in the control, whereas the sample composition containing N,N-dimethylacetamide and 2-fluoroacryloyl fluoride was a transparent liquid and no change in the characteristics was observed.

This confirmed that N,N-dimethylacetamide stabilizes 2-fluoroacryloyl fluoride.

### Example 2

Each sample was prepared by adding N,N-dimethylformamide in an amount specified in Table 1 per 100 mass % of 2-fluoroacrylic acid methyl ester.

The samples were placed in individual sample bottles. Each bottle was closed with a cap and allowed to stand for 5 hours at 60°C. Thereafter, the characteristics of each sample were observed. The results revealed that no change in characteristics was observed in any sample until after 3 hours. However, thereafter, the viscosity increased in 2-fluoroacrylic acid methyl ester in which N,N-dimethylformamide was not added (sample 2-1)(control), and this sample was completely solidified after 5 hours. In contrast, the characteristics of the sample compositions containing N,N-dimethylformamide and 2-fluoroacrylic acid methyl ester (sample 2-2 and sample 2-3) were not changed even after 5 hours.

This confirmed that N,N-dimethylacetamide stabilizes 2-fluoroacrylic acid methyl ester.

**Table 1**

| Sample | N,N-dimethylformamide (mass %) |
|---|---|
| 2-1 | 0.0 |
| 2-2 | 3.0 |
| 2-3 | 5.0 |

## Claims

1. A composition comprising
(A) an acrylic acid derivative of formula (I): 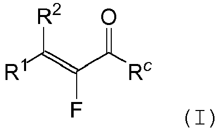 wherein, R¹ and R² each independently are H, halogen, alkyl, fluoroalkyl or optionally substituted aryl; and R*^{c}* is F, Br, I, or -OR³ wherein R³ is H, alkyl, fluoroalkyl or optionally substituted aryl; and
(B) amide,
wherein the content of the acrylic acid derivative (A) in the composition is ≥ 30% (w/w).

2. The composition of claim 1, wherein the amide (B) is C₁₋₆-amide.

3. The composition of claim 2, wherein the amide (B) is N,N-dimethylformamide or N,N-dimethylacetamide.

4. The composition of any of claims 1-3, wherein R¹ is H, C₁₋₂₀-alkyl or C₁₋₂₀-fluoroalkyl.

5. The composition of any of claims 1-4, wherein R¹ is H.

6. The composition of any of claims 1-5, wherein R² is H, C₁₋₂₀-alkyl or C₁₋₂₀-fluoroalkyl.

7. The composition of any of claims 1-6, wherein R² is H.

8. The composition of any of claims 1-7, wherein R³ is linear C₁₋₂₀-alkyl.

9. A method for stabilizing (A) an acrylic acid derivative of formula (I): 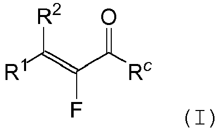 wherein, R¹ and R² each independently are H, halogen, alkyl, fluoroalkyl or optionally substituted aryl; and R*^{c}* is F, Br, I, or -OR³ wherein R³ is H, alkyl, fluoroalkyl or optionally substituted aryl;
comprising making the acrylic acid derivative of formula (I) coexist with amide.

## Patentansprüche

1. Zusammensetzung, umfassend
(A) ein Acrylsäurederviat der Formel (I): 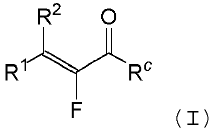 worin R¹ und R² jeweils unabhängig H, Halogen, Alkyl, Fluoralkyl oder gegebenenfalls substituiertes Aryl sind und R^{c} F, Br, I oder -OR³ ist, wobei R³ H, Alkyl, Fluoralkyl oder gegebenenfalls substituiertes Aryl ist, und
(B) Amid,
wobei der Gehalt des Acrylsäurederivats (A) in der Zusammensetzung ≥ 30 % (Gew/Gew) beträgt.

2. Zusammensetzung gemäß Anspruch 1, wobei das Amid (B) C₁₋₆-Amid ist.

3. Zusammensetzung gemäß Anspruch 2, wobei das Amid (B) N,N-Dimethylformamid oder N,N-Dimethylacetamid ist.

4. Zusammensetzung gemäß irgendeinem der Ansprüche 1-3, wobei R¹ H, C₁₋₂₀-Alkyl oder C₁₋₂₀-Fluoralkyl ist.

5. Zusammensetzung gemäß irgendeinem der Ansprüche 1-4, wobei R¹ H ist.

6. Zusammensetzung gemäß irgendeinem der Ansprüche 1-5, wobei R² H, C₁₋₂₀-Alkyl oder C₁₋₂₀-Fluoralkyl ist.

7. Zusammensetzung gemäß irgendeinem der Ansprüche 1-6, wobei R² H ist.

8. Zusammensetzung gemäß irgendeinem der Ansprüche 1-7, wobei R³ geradkettiges C₁₋₂₀-Alkyl ist.

9. Verfahren zur Stabilisierung (A) eines Acrylsäurederivats der Formel (I): 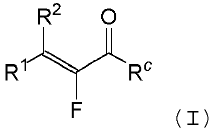 worin R¹ und R² jeweils unabhängig H, Halogen, Alkyl, Fluoralkyl oder gegebenenfalls substituiertes Aryl sind und R^{c} F, Br, I oder -OR³ ist, wobei R³ H, Alkyl, Fluoralkyl oder gegebenenfalls substituiertes Aryl ist,
umfassend das Veranlassen, dass das Acrylsäurederivat der Formel (I) zusammen mit dem Amid vorliegt.

## Revendications

1. Composition comprenant
(A) un dérivé d'acide acrylique de formule (l) : 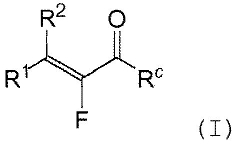
dans laquelle, R¹ et R² sont chacun indépendamment H, un halogène, un alkyle, un fluoroalkyle ou un aryle facultativement substitué ;
et R*^{c}* est F, Br, I, ou -OR³ dans lequel R³ est H, un alkyle, un fluoroalkyle ou un aryle facultativement substitué ; et
(B) un amide,
dans laquelle la teneur en dérivé d'acide acrylique (A) dans la composition est ≥ 30 % (poids/poids).

2. Composition selon la revendication 1, dans laquelle l'amide (B) est un amide en C₁₋₆.

3. Composition selon la revendication 2, dans laquelle l'amide (B) est un N,N-diméthylformamide ou un N,N-diméthylacétamide.

4. Composition selon l'une quelconque des revendications 1 à 3, dans laquelle R¹ est H, un alkyle en C₁₋₂₀ ou un fluoroalkyle en C₁₋₂₀.

5. Composition selon l'une quelconque des revendications 1 à 4, dans laquelle R¹ est H.

6. Composition selon l'une quelconque des revendications 1 à 5, dans laquelle R² est H, un alkyle en C₁₋₂₀ ou un fluoroalkyle en C₁₋₂₀.

7. Composition selon l'une quelconque des revendications 1 à 6, dans laquelle R² est H.

8. Composition selon l'une quelconque des revendications 1 à 7, dans laquelle R³ est un alkyle en C₁₋₂₀ linéaire.

9. Procédé de stabilisation d'(A) un dérivé d'acide acrylique de formule (l) : 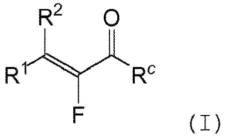
dans laquelle, R¹ et R² sont chacun indépendamment H, un halogène, un alkyle, un fluoroalkyle ou un aryle facultativement substitué ;
et R*^{c}* est F, Br, I, ou -OR³ dans lequel R³ est H, un alkyle, un fluoroalkyle ou un aryle facultativement substitué ;
comprenant l'étape consistant à faire coexister le dérivé d'acide acrylique de formule (l) avec l'amide.
